# NEW EUROPEAN PATENT SPECIFICATION

(11) **EP 0 460 041 B2**
(45) Date of publication and mention of the opposition decision: **25.08.2004**
(45) Mention of the grant of the patent: 08.06.1994
(21) Application number: 90903862.2
(22) Date of filing: 22.02.1990
(51) Int. Cl.: C12Q 1/68, C07H 21/04, C07K 14/35, C07K 7/00

(54) **PROBES, KITS AND METHODS FOR THE DETECTION AND DIFFERENTIATION OF MYCOBACTERIA**
SONDEN, AUSRÜSTUNGEN UND VERFAHREN ZUM NACHWEIS UND ZUR DIFFERENZIERUNG VON MYCOBAKTERIEN
SONDES, KITS ET PROCEDES DE DETECTION ET DE DIFFERENCIATION DE MYCOBACTERIES

(30) Priority: 22.02.1989 GB 8903968; 09.01.1990 GB 9000411
(43) Date of publication of application: 11.12.1991
(73) Proprietor: Cogent Limited, London, EC4N 4TP (GB)
(72) Inventor: McADAM, R. A., Dept. of Micr. & Imm., 1300 Morris Park Av., Bronx, NY 10461 (US); CATTY, D., Dept. of Imm. & Micr., Birmingham B15 2TJ (GB); DALE, Jeremy, Watson, Surrey GU1 3LA (GB); ZAINUDDIN, Zainul, Fadziruddin, bin, Penang 11-800 (MY)
(74) Representative: Stuart, Ian Alexander
(86) International application number: PCT/GB1990/000276
(87) International publication number: WO 1990/010085

(56) References cited:
- WO-A1-91/03558
- JOURNAL OF GENERAL MICROBIOLOGY, vol. 135, no. 9, September 1989, SGM (GB); Z.F. ZAINUDDIN et al., pp. 2347-2355#
- JOURNAL OF CLINICAL MICROBIOLOGY, vol. 26, no. 11, November 1988,American Society for Microbiology; K.D. EISENACH et al., pp. 2240-2245#
- NUCLEIC ACIDS RESEARCH, vol. 18, no. 1, January 1990, Oxford University Press (GB); D. THIERRY et al., p. 188#
- BIOLOGICAL ABSTRACTS, vol. 87, 1989, Philadelphia, PA (US); R.P.PRABHAKARA et al., no. 103147#
- EMBL Accesion Number X17348

## Description

### TECHNICAL FIELD

The present invention relates to gene probes, kits and methods for the detection and differentiation of Mycobacteria. In particular, the present invention relates to gene probes, kits and methods for the diagnosis of tuberculosis and/or for epidemiological study tools for investigating the progress of infections caused by members of the M.tuberculosis complex.

### BACKGROUND ART

In some developed countries including the United Kingdom, tuberculosis is numerically one of the major notifiable infectious diseases and yet the mechanism of pathogenicity of M.tuberculosis is poorly understood. In the developing or 'third' world, this disease is an endemic health problem of vast proportions and therapy involves long periods of treatment with combinations of antibiotics. It is well recognised that one of the major problems in tackling tuberculosis is the lack of a simple, reliable and robust serodiagnostic or gene probe assay. These are necessary because current diagnostic tests, even those available in technically advanced rich nations, are poorly specific and insensitive, being based on a combination of relatively crude symptomology and radiography, staining for acid fast bacilli and bacterial culture. The first two are widely variable features and the second two are notoriously unreliable. In particular, with presently available tests, several weeks may be required to obtain a definite result and the detection of small numbers of M.tuberculosis bacteria in heavily contaminated samples is often difficult. The specific identification of Mycobacteria is also difficult, and especially the differentiation between the members of the M.tuberculosis complex: M.tuberculosis itself, the bovine strain M.bovis, M.africanum, M.microti and the vaccine strain BCG (which may cause disease in immunologically suppressed individuals. Many attempts have been made to develop new laboratory tests for tuberculosis but all have suffered from poor specificity and/or sensitivity. Gene probes for specific DNA sequences of the organism can detect small amounts of Mycobacterial genome reliably, by procedures that do not require a prolonged culture step or the laborious examination by trained staff of stained sputum smears. Gene probe analysis offers a sensitive method for the rapid detection of small numbers of specific bacteria in the presence of other organisms.

As well as being a significant health problem in humans, infections caused by Mycobacteria are also a significant health problem in cattle, deer, sheep and badgers and the probes provided herein are also useful for diagnostic/epidemiological study tools for use in respect of these species.

Gene probes for identifying strains of the M.tuberculosis complex are commercially available, but depend on detecting ribosomal RNA and require the bacteria to be cultivated first. Although these gene probes are capable of identifying the M.tuberculosis complex, they are not suitable for detecting bacteria in a specimen of sputum. The cultivation step also increases the test time and this is disadvantageous.

Described herein is the isolation and cloning of a fragment of M.tuberculosis DNA containing a repetitive element specific to the M.tuberculosis complex. This fragment hybridises to multiple polymorphic restriction fragments in different isolates of M.tuberculosis and is therefore able to fingerprint isolates for studies of transmission of tuberculosis. Only a few hybridising bands are detected in digests of M.bovis or BCG DNA, and the probe therefore has the unique ability to distinguish rapidly between these different members of the M.tuberculosis complex.

Several repetitive elements have been isolated from Mycobacterial species, including one from M.leprae (Clark-Curtiss, J.E. & Walsh, G.P. (1989) Journal of Bacteriology 171, 4844-4851; Clark-Curtiss, J.E. & Docherty, M.A. (1989) Journal of Infectious Diseases 159, 7-15; and Grosskinsky, C.M. Jacobs, W.R. Clark-Curtiss, J.E. & Bloom, B.R. (1989) Infection and Immunity 57, 1535-1541) and the insertion sequence IS900 from M.paratuberculosis (Green, E.P. Tizard, M.L.V. Moss, M.T. Thompson, J,, Winterbourne, D.J., McFadden, J.J. & Hermon-Taylor, J. (1989) Nucleic Acids Research 17, 9063-9072). However, these repetitive elements are both species-specific and appear to give a constant hybridisation pattern with strains from different sources.

This application describes the characterisation and sequence analysis of a repetitive element, which identifies it as a member of the IS3 family of insertion sequences, of which several members have previously been characterised from species of the Enterobacteriaceae.

It has now been found that DNA probes having potential applications to the general diagnosis of Mycobacteria and to the specific diagnosis of tuberculosis can be derived from deoxyribonucleotide sequences capable of hybridizing with those sequences present in a naturally occurring plasmid.

As part of an investigation into antibiotic resistance, the presence of plasmids in M.tuberculosis was sought by hybridizing the total DNA from three clinical isolates with DNA from a naturally occurring plasmid known to exist in M.fortuitum (A. Labidi, C. Dauguet, K.S. Goh & H.L. David, 1984. Plasmid profiles of Mycobacterium fortuitum complex isolates. Current Microbiology 11: 235-240). Plasmids have not hitherto been found in M.tuberculosis, and it was hoped that they would be revealed by the use of the M.fortuitum plasmid DNA as a probe. Surprisingly, while this did not reveal the presence of any plasmids in M.tuberculosis, it did show that there are M.tuberculosis chromosomal DNA fragments which can hybridize with the plasmid DNA. Moreover, in total DNA from the three clinical isolates digested with restriction endonucleases BamHI or PvuII, the size of the hybridising fragments was not the same for each strain.

Gene probes for the detection of Mycobacterial infection can have varying degrees of specificity depending on how unique the gene sequences they detect in a bacterial genome, are to a given family, genus, species or strain. Probes of different specificities can be of use depending on the clinical analysis required. Thus, one probe could detect a sequence pattern that is found in many different species (e.g; M.tuberculosis and M.bovis) within a given genus (e.g; Mycobacterium). In other cases. gene probes may be specific for a particular species, and even for different strains of that species.

This varying specificity of gene probes has a practical use. For example, as a first line of diagnosis it may be more appropriate to use a probe which detected general Mycobacterial infection and then, if necessary use fine-tuning probes to diagnose which species of Mycobacteria are involved.

### DISCLOSURE OF INVENTION

The present invention provides a nucleotide probe for the diagnosis and/or epidemiological study of Mycobacterial infection, which hybridises with M.tuberculosis genomic DNA obtainable by screening a M.tuberculosis genomic library with DNA of a plasmid of M.fortuitum.

The present invention also provides a nucleotide probe for the diagnosis and/or epidemiological study of Mycobacterial infection, which hybridises with genomic DNA of M.tuberculosis and with DNA of a plasmid of M.fortuitum.

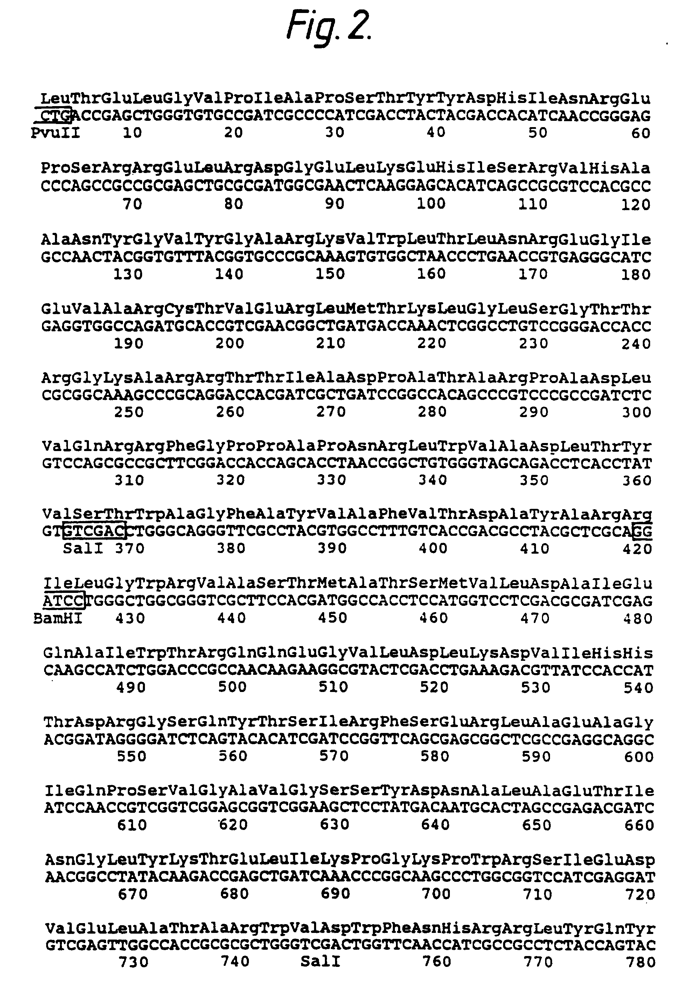

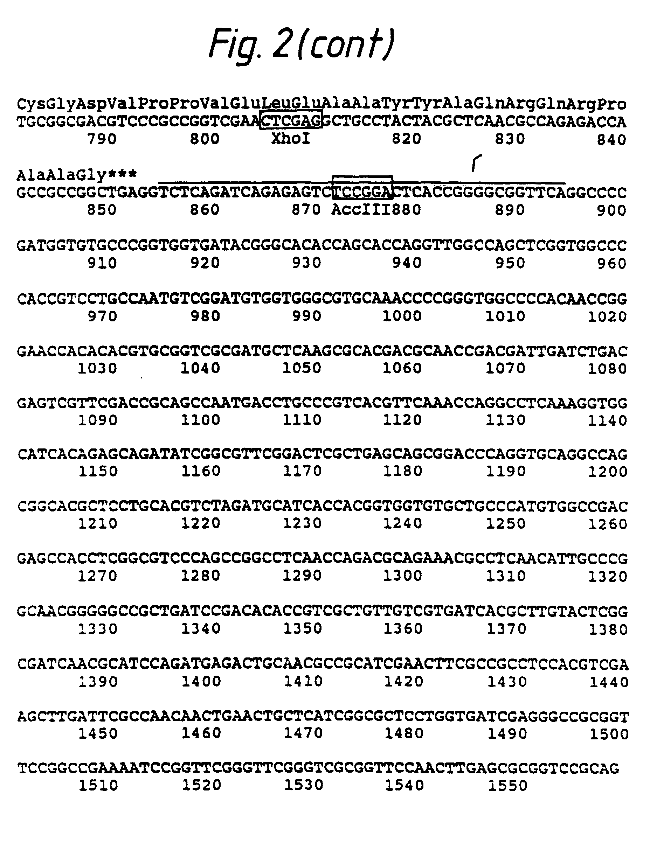

We disclose a nucleotide probe for the diagnosis and/or epidemiological study of Mycobacterial infection, which comprises, or hybridises with, the nucleotide sequence depicted in Fig. 2 hereof or its complementary sequence, or which comprises or hybridises with a nucleotide sequence obtainable from a genomic library of an organism of the M.tuberculosis complex, by hybridisation with the nucleotide sequence depicted in Fig. 2 hereof, and which is capable of distinguishing and characterising bacterial members of the M.tuberculosis complex either from each other, or from other bacteria not of the complex.

Also provides by the invention is a nucleotide probe for the diagnosis and/or epidemiological study of Mycobacterial infection, wherein the genomic library is obtainable from M.tuberculosis strain 50410.

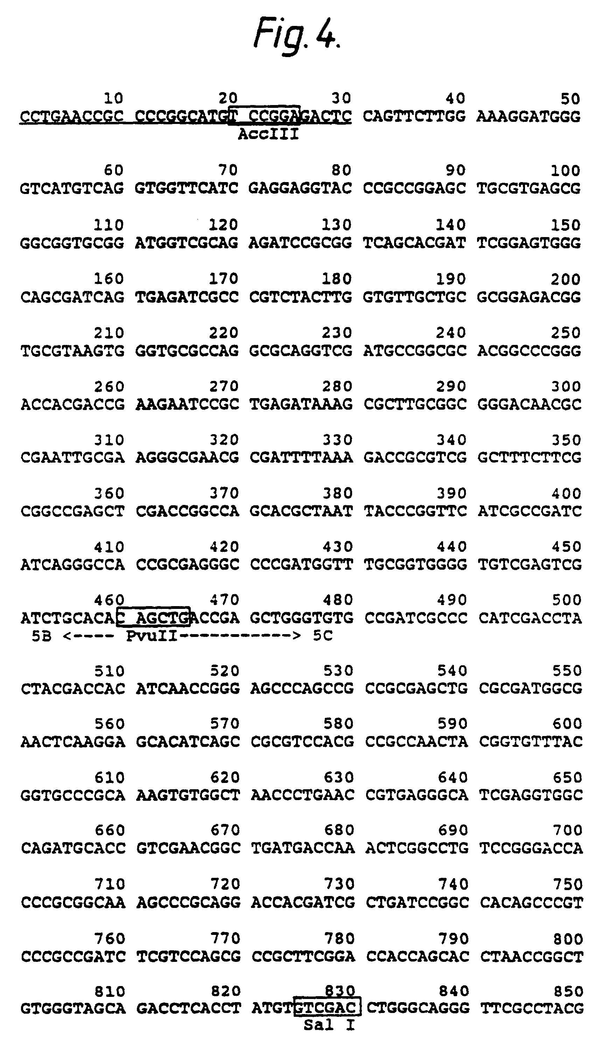

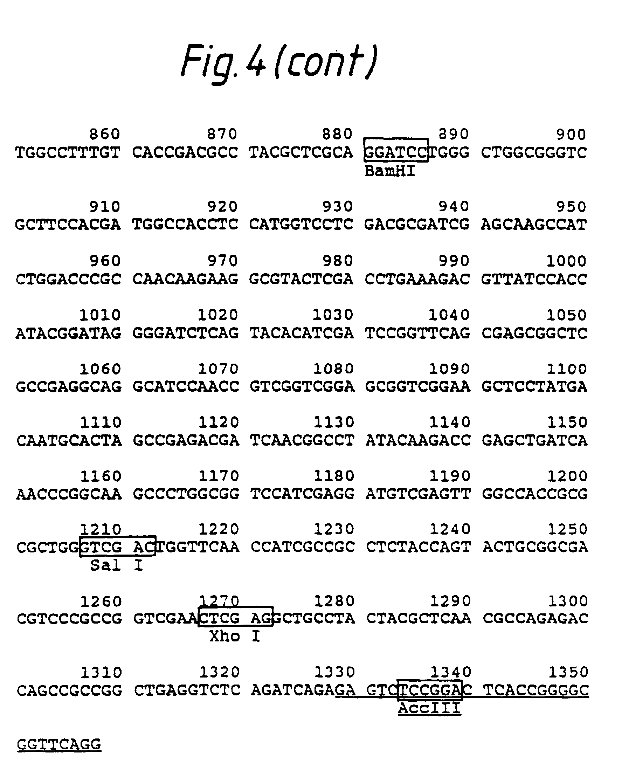

We also disclose a nucleotide probe for the diagnosis and/or epidemiological study of Mycobacterial infection which comprises, or hybridises with, part or all, of the nucleotide sequence shown in either Fig.2 or Fig.4 of the drawings or its complementary sequence.

The nucleotide probe may comprise or hybridise with part or all of an insertion element nucleotide sequence which in the genome of M.tuberculosis strain 50410 is bounded by two inverted repeat sequences and contains the nucleotide coding sequence identified in Fig. 2 of the drawings.

Also provided by the present invention is a nucleotide probe for the diagnosis and/or epidemiological study of Mycobacterial infection which comprises or hybridises with a flanking sequence of nucleotides which in the genome of M.tuberculosis strain 50410 occur adjacent to an insertion element nucleotide sequence, bounded by two inverted repeat sequences and containing the nucleotide coding sequence identified in Fig. 2 of the drawings.

For example, the nucleotide probe may comprise or hybridise with part or all of the flanking sequence of nucleotides which occurs downstream of the 3' end of base 896 in Fig.2 of the drawings.

The present invention also provides a nucleotide probe for the diagnosis and/or epidemiological study of Mycobacterial infection which comprises, or hybridises with, part or all of an approximately 1.9kb nucleotide sequence which, in the genome of M.tuberculosis strain 50410, occurs immediately downstream of the 3' end of the sequence shown in Fig.2 of the drawings.

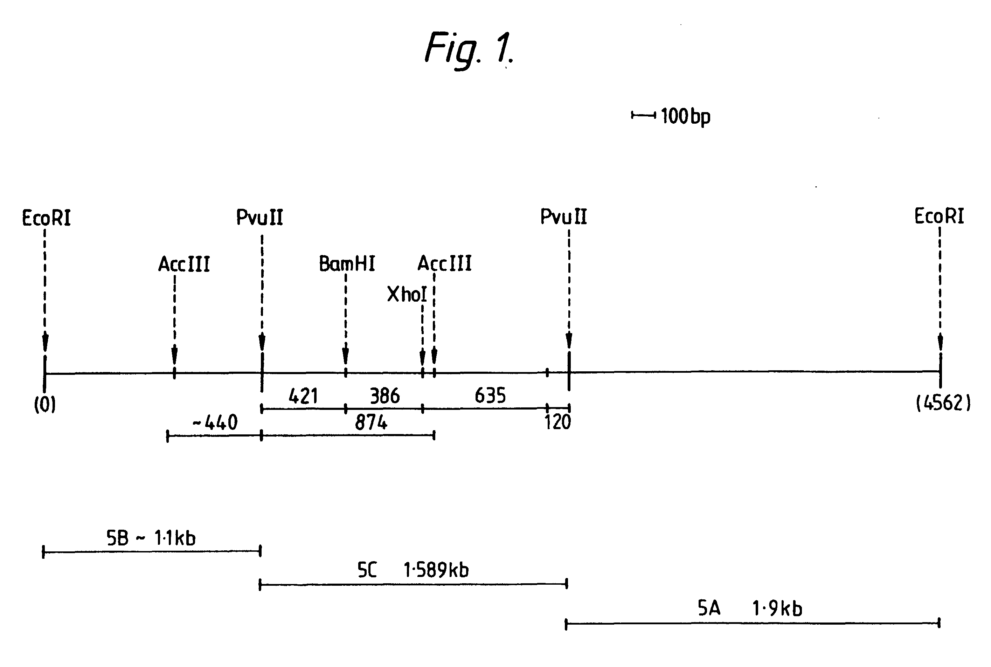

We also disclose a nucleotide probe for the diagnosis and/or epidemiological study of Mycobacterial infection, which comprises or hybridises strongly with part or all of a nucleotide sequence which occurs in the genome of M.tuberculosis strain 50410 and which is characterised by the restriction map as shown in Fig.1 of the drawings.

The nucleotide probe disclosed herein may be used for the diagnosis of and/or epidemiological study of Mycobacterial infection. The nucleotide probes may be able to distinguish between different strains of M.tuberculosis. The nucleotide probes may be able to distinguish between M.tuberculosis, M.bovis and BCG. The nucleotide probes may not show significant hybridisation with M.paratuberculosis, M.intracellulare, M.scrofulaceum, M.phlei, M.fortuitum, M.chelonei, M.kansasii, M.avium, M.malnioense, M.flavescens and M.gordonae.

The nucleotide probes may be used for the detection of Mycobacteria in clinical samples by the techniques of dot blot analysis, solution hybridization, Southern blot analysis or polymerase chain reaction. The clinical samples may comprise sputum, urine, cerebrospinal fluid, tissue samples, blood and other body fluids.

The present invention also comprises diagnostic kits comprising the nucleotide probes as claimed herein.

The present invention also provides a method for detecting, distinguishing and/or characterising Mycobacteria in clinical samples for the purposes of epidemiological study which comprises using the nucleotide probes as claimed herein.

The present invention also provides methods for the production of said nucleotide probes.

The present invention also provides a method for distinguishing and characterising bacterial members of the M.tuberculosis complex, both from each other and from other bacteria not of the complex, which method comprises: i) digesting DNA from a sample of bacteria with a particular restriction enzyme; and ii) carrying out hybridisation analysis using nucleotide probe as claimed herein.

The nucleotide sequence comprising the gene probe may not necessarily contain a restriction site for the restriction enzyme.

### BRIEF DESCRIPTION OF DRAWINGS

In order that the present invention is more clearly understood, embodiments will be described in more detail by way of example only and with reference to the figures wherein:
Fig. 1 shows a restriction map of probe 5;
Fig. 2 shows the DNA sequence of fragment 5C from probe 5 and the translation product of the large open reading frame;
Fig. 3 shows a comparison of primary DNA structure of part of 5C compared with the insertion sequences IS3 and IS3411 of E.coli;
Fig. 4 shows the DNA sequence overlapping part of fragment 5B and part of fragment 5C of probe 5, namely the insertion sequence (IS986) from the 5kb DNA fragment of M.tuberculosis;
Fig. 5 shows the location of designated open reading frames;
Fig. 6 shows the alignment of potential translated product of IS986 ORFb with putative transposases of other IS3-like elements;
Fig. 7 shows the alignment of potential translated products of ORFa1 and ORFa2 with corresponding regions of other IS3-like elements;
Fig. 8 shows a comparison of the inverted repeat ends of ISTB and IS3411;
Fig. 9 shows the alignment of the potential translated products of the large open reading frames of 5C and IS3411;
Fig. 10 shows the alignment of the potential translated products of the large open reading frames of 5C and IS3;
Fig. 11 shows the alignment of the potential translated products of the large open reading frames of 5C, IS3411 and IS3;
Fig. 12 shows the alignment of the potential translated products of the large open reading frames of the insertion sequence (IS986) from the 5kb DNA fragment of M.tuberculosis with those of IS3411 and IS3;
Fig. 13 shows the alignment of the potential translated products of the large open reading frames of the insertion sequence (IS986) from the 5kb DNA fragment of M.tuberculosis with those of IS3411 and IS3 wherein the C-terminal region of the IS3411 sequence (IS3411') is read from the -1 frame with respect to the rest of the IS3411 sequence;
Fig. 14 shows a restriction map of probe 9; and
Fig. 15 shows diagrammatically the relationship between probes 5 and 12J-B.

### MODES FOR CARRYING OUT THE INVENTION

### Probes 9 and 5

As part of an investigation into the possible presence of plasmids in clinical isolates of M.tuberculosis, total DNA from three such isolates was subjected to Southern blotting and probed with a naturally occurring plasmid from M.fortuitum. This plasmid, referred to as pUS300, was obtained from M.fortuitum strain CIPT 14-041-0003 in the Collection de I'Institut Pasteur, Tuberculose, Paris, France (deposited at the National Collection of Type Cultures, Colindale, London UK NW9 5HT under accession number NCTC 12381 on 21 February 1990). The results showed that there were chromosomal DNA fragments in the strains of M.tuberculosis which were capable of hybridizing to this M.fortuitum plasmid, and also that in material digested with BamHI or PvuII, the size of the hybridizing fragments were not the same for each strain.

In order to isolate these hybridizing fragments, a total DNA library from a clinical isolate of M.tuberculosis (strain 50410, obtained from the Public Health Laboratory, Dulwich, London, England) was constructed in the lambda phage vector EMBL4 by ligation of a partial Sau3AI digest of the M.tuberculosis DNA with BamHI-digested EMBL4. The library was screened with a DNA probe derived by labelling a recombinant plasmid pUS301. This plasmid was constructed by ligating an EcoRI digest of plasmid pUS300 with an EcoRI digest of the E.coli plasmid vector pUC19. Positive plaques were purified through further rounds of plaque screening. The probes described below are obtained from the recombinant phage, referred to as the EMBL4/A-3 clone (deposited at the National Collection of Type Culture, Colindale, London UK NW9 5HT under accession number NCTC 12380 on 21 February 1990), of one of the positive plaques obtained by this procedure.

The DNA from this recombinant phage EMBL4/A-3 clone was extracted and digested with EcoRI. Agarose gel electrophoresis and Southern blotting demonstrated that EcoRI-digested EMBL4/A-3 contained a series of fragments including one of approximate size 9000 base pairs (9kb) and one of approximate size 5000 base pairs (5kb) which hybridized with the plasmid pUS300. These fragments were each excised from the gel and are referred to as probe 9 (the 9 kb fragment) and probe 5 (the 5 kb fragment) respectively. Isolation of the probe 5 by hybridisation with M.fortuitum plasmid pUS300 is more fully described in Zainuddin and Dale; J. Gen. Micro. (1989) 135, pp 2347-2355.

The 5kb EcoRI fragment from the lambda clone A3 (Zainuddin, Z.F. & Dale, J.W. (1989) Journal of General Microbiology 135, 2347-2355) was cloned using the plasmid vector pAT153 to generate plasmid pRP5000. Digestion of the insert fragment from pRP5000 with PvuII generated three fragments designated 5A, 5B and 5C (Fig. 1) which were converted to blunt-ended fragments and ligated with PvuII digested pAT153, generating plasmids pRP5100, pRP5200 and pRP5300 respectively.

Specific subfragments from pRP5000, pRP5200 and pRP5300 generated using BamHI, XhoI, HindIII and Sa1I (Fig. 1) were cloned in M13mp18 and M13mp19 using the M13 Cloning Kit (New England Biolabs). The smaller EcoRI-BamHI fragment from pRP5000 was cloned into Bluescript pKS and nested deletions were carried out using the Erase-a-Base technique (Promega). Sequencing was performed with Taq and T7 polymerases (Promega) and Sequenase Version 2 (US Biochemicals), using the 370 Automated Sequencer (Applied Biosystems). Fragments with overlaps of at least 50bp were sequenced in both directions.

Searches of GenBank, EMBL, NBRF and SwissProt databanks were carried out using the SEQ-NET node at the SERC facility at Daresbury, by means of the UWGCG package and WordSearch program (Devereux, J., Haeberli, P. & Smithies, O. (1984) Nucleic Acids Research 12, 387-395; and Wilbur, W.J. & Lipman, D.J. (1983) Proceedings of the National Academy of Sciences USA 80, 726-730) and the NAQ program from the Protein Identification Resource. Sequence analyses were performed with the Staden-Plus package (Amersham) using DIAGON (Staden, R. (1982) Nucleic Acids Research 10, 2951-2961) for sequence comparisons and both Positional Base Preference (Staden, R. (1984) Nucleic Acids Research 12, 551-567) and Shepherd RNY (Shepherd, J.C.W. (1981) Proceedings of the National Academy of Sciences USA 78, 1596-1600) methods for identification of reading frames, supplemented by the use of codon preference analysis (Staden, R. & McLachlan, A.D. (1982) Nucleic Acids Research 10, 141-156) based on a table of preferred codon usage in M.tuberculosis (Dale, J.W. and Patki, A. (1990) in The Molecular Biology of Mycobacteria (McFadden, J.J. Ed.) in press). Multiple sequence alignments were carried out with the CLUSTAL software (Higgins, D.C. & Sharp, P.M. (1988) Gene 73, 237-244) supplemented by manual adjustment.

### Probe 9

Probe 9 was radioactively labelled with ³²P using the Multiprime Random Primer Extension method (Amersham) and hybridized to Southern blots of PvuII-digested total DNA from eight clinical isolates of M.tuberculosis (isolate number 50410, 60925, 61066, 61104, 61125, 61267, 61377, 61513) as well as M.bovis (field strain, Central Veterinary Laboratory) and BCG (NCTC 5692). After agarose gel electrophoresis, the DNA fragments were transferred to a Hybond-N filter and fixed by baking at 80°C for 1 hour. The filter was prehybridized at 68°C in hybridisation buffer. Hybridisation with the probe was carried out in the same buffer at 68°C overnight.

The hybridization buffer consisted of 5X Denhardt's solution, 5X SSPE buffer, 0.2% sodium dodecyl sulphate (SDS) and 100 µg./ml. sonicated salmon sperm DNA. The Denhardt's solution and the SSPE buffer were made up as stock solutions as follows:
50X Denhardt's solution: 0.5g. Ficoll™ (mw 400,000), 0.5g. polyvinylpyrrolidone (mw 40,000), 0.5 g. bovine serum albumin, were dissolved in sterile deionised distilled water to a final volume of 50mls which was dispensed into aliquots and stored at -20°C.
20X SSPE buffer: 3.6M NaCl, 20mM ethylenediaminetetra-acetic acid (EDTA), 0.2M NaH₂PO₄/Na₂HPO₄, pH 7.7 were dissolved in deionized distilled water and autoclaved.

The filter was then washed twice with 2X SSC, once with 2X SSC containing 0.1% SDS and once with 0.1X SSC containing 0.1% SDS. All washes were done at 68 °C. The SSC was made up as a stock solution as follows:
20X SSC: 3M NaCl, 0.3M sodium citrate were dissolved in distilled water and autoclaved after the pH had been adjusted to 7.0.

The filter was covered with Saran wrap and exposed to X-ray film (RX, Fuji) for 16 hours at room temperature.

Each strain of M.tuberculosis hybridized to probe 9 exhibited several hybridizing bands; some elements of this pattern varied from strain to strain while others remained constant. M.bovis and BCG also hybridized to probe 9 with a pattern which retained the conserved features of the M.tuberculosis pattern.

The following species of Mycobacteria (one strain each except where indicated) did not hybridize with probe 9 to any significant extent: M.paratuberculosis, M.intracellulare, M.scrofulaceum, M.phlei, M.fortuitum (three strains), M.kansasii, M.avium, M.malnioense, M.flavescens, M.gordonae and M.chelonei (two strains).

Probe 9 was, therefore, specific for the M.tuberculosis complex (which includes M.bovis and BCG), with some ability to differentiate between strains.

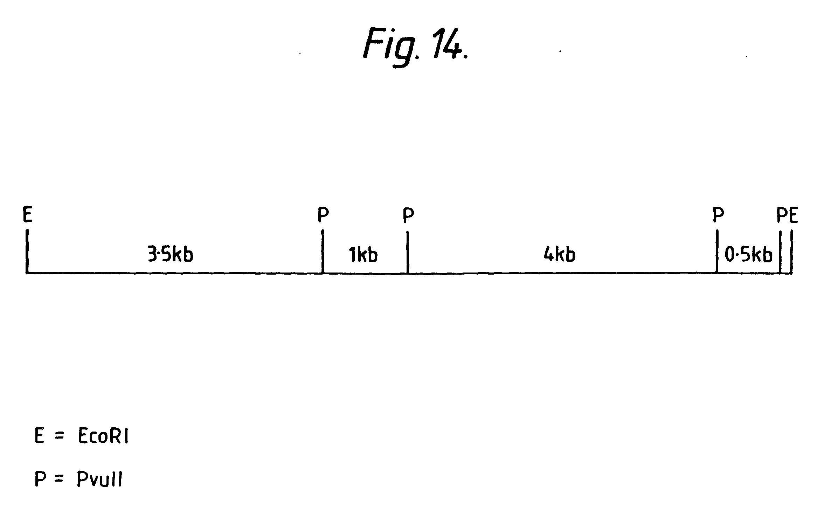

A restriction map of probe 9 is shown in Fig. 14. The probe is bound by two EcoRI sites and divided by four internal PvuII sites into fragments of approximately 3.5kb, 1kb, 4kb and 0.5kb.

### Probe 5

Studies on probe 5 have revealed that it comprises a sequence which encodes an insertion element (designated IS986) which appears to be present in a variable number of copies (up to about 15) in M.tuberculosis, M.bovis, M.africanum, M.microti and M.bovis BCG of the M.tuberculosis complex. The insertion element has been compared to the previously described insertion elements IS 3 and IS 3411 found in E.coli. The insertion element of probe 5 has close homology to IS 3411 which probably encodes a transposase.

A restriction map of probe 5 is shown in Fig. 1. The probe can be divided at two PvuII sites into fragments 5A, 5B and 5C as shown.

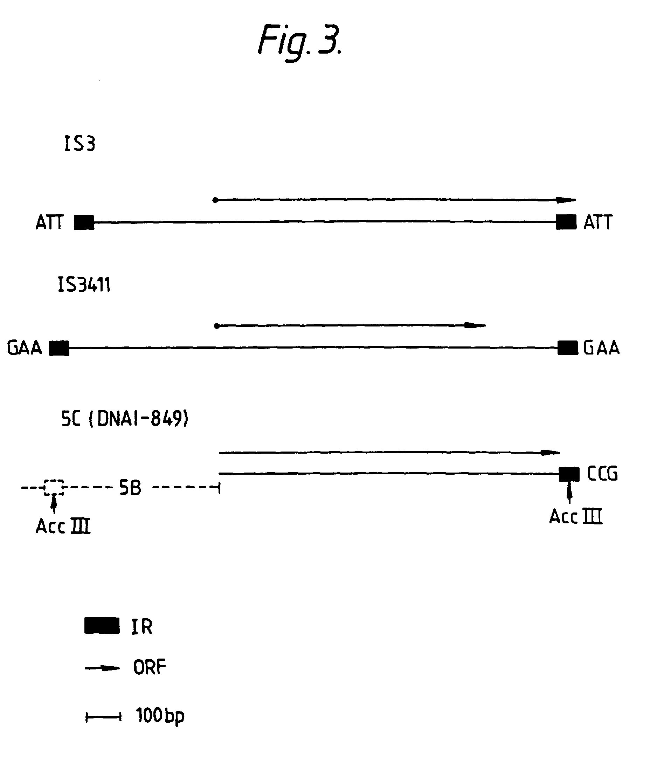

The sequence of 5C is shown in Fig. 2. Useful restriction sites are boxed and a sequence with 29/40 identity with the right-hand inverted repeat (IR) from IS 3411 and 20/40 with the inverted repeat from IS 3 is underlined (Ishiguro & Sato 1988, J. Bacteriology 170, 1902-1906; Timmerman & Yu 1985, Nucl. Acids Res. 13, 2127-2139). Line diagrams comparing the primary DNA structure of part of 5C compared with IS 3 and IS 3411 are shown in Fig. 3.

Fig.4 shows a DNA sequence which overlaps part of fragment 5B and part of fragment 5C of probe 5. As in Fig.2 useful restriction sites are boxed. The PvuII restriction site defines the join between fragments 5B and 5C. This DNA sequence comprises two inverted repeat sequences (27/30 bases matching) which have been underlined in Fig.4. The left-hand inverted repeat CCTGAACCGCCCCGG CATGTCCGGAGACTC is located within fragment 5B to the 5' side of a first Acc III site, whilst the right-hand inverted repeat GAGTCTCCGGACTCACCGGGGCGGTTCAGG is located within fragment 5C to the 3' side of a second Acc III site. The sequence between these inverted repeat sequences comprises the insertion element IS986 (of approximately 1358 bp) which is present in a variable number of copies in members of the M.tuberculosis complex.

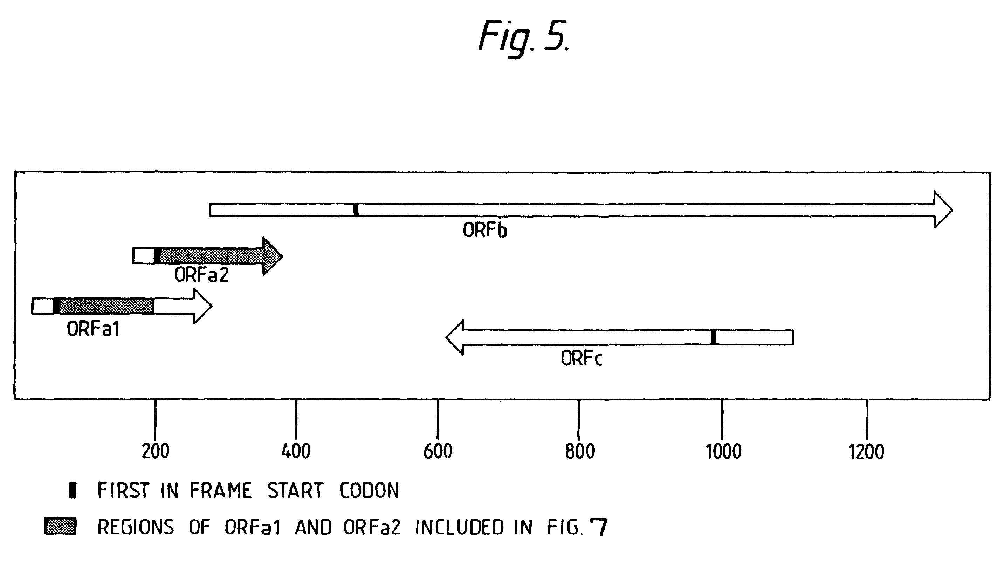

Examination of the insertion element revealed one long open reading frame (ORFb: bases 274 to 1311) with a potential translational start codon (GUG) at position 478, and another (ORFc) in the reverse direction (1107 to 622) (Fig. 5). Positional base preference analyses indicated both of these as potentially translated regions, together with parts of two shorter ORFs (6 to 275 and 164 to 376). (For reasons discussed below, the latter two are considered together and designated ORFa1 and ORFa2 respectively; the regions likely to be translated are indicated in Fig. 5. The codon usage of ORFb, and to a lesser extent ORFc, is consistent with the high degree of codon bias normally shown by mycobacterial genes (Dale, J.W. and Patki, A. (1990) in The Molecular Biology of Mycobacteria (McFadden, JJ., Ed.) in press). This was also true of the indicated regions of ORFa1 and ORFa2 (Fig. 5), although not for the remainder of these ORFs (see below)).

The sequence of the hypothetical translation product of ORFb was screened against the NBRF and SwissProt databanks. One sequence was identified with homology significantly above background, which was the putative transposase protein of the insertion sequence IS3411, from E.coli - (Ishiguro and Sato; 1988, J. Bacteriology 170, 1902-1906); a lower degree of similarity was seen with hypothetical proteins translated from the sequences of two other insertion sequences, IS600 and IS629, both from Shigella sonnei (Matsutani, S., Ohtsubo, H., Maeda, Y. & Ohtsubo, E. (1987) Journal of Molecular Biology 196, 445-455). All these sequences belong to the IS3 family.

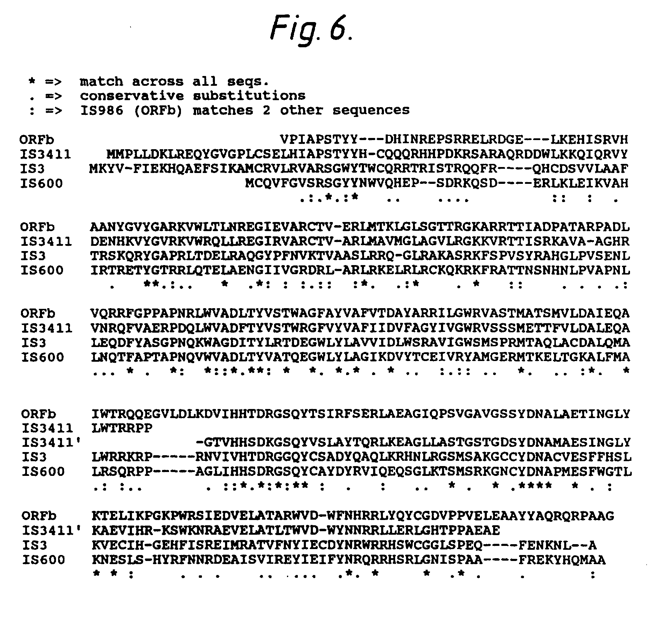

A multiple alignment of these sequences, and that of the IS3 transposase (Timmerman, K.P. & Tu, C-P.D. (1985) Nucleic Acids Research 13, 2127-2139), demonstrates a marked degree of resemblance except for the C-terminal portion of the IS3411 protein. The different structure of this region of IS3411 is also evident from the alignment of the putative transposases (proteins which allow the DNA segment comprising the insertion element bound by inverted repeats, to excise and insert at another part of the genome), of IS3 and IS3411 as shown by Ishiguro & Sato 1988. However, a comparison of the products of all three reading frees of the complete sequences of IS3, IS3411 and IS986 showed homology of the C-terminal portion of the IS986 ORFb with the -1 frame of IS3411. A multiple alignment, using an IS3411 product with a hypothetical frameshift (Fig. 6) (the sequences were split at the point corresponding to the putative frameshift in IS3411; the two portions were aligned separately and re-combined manually. IS3411' is read from the -1 frame with respect to the first part of the sequence), shows that 27% of the amino acid residues of the IS986 ORFb product are also present in at least two of the other three sequences used for comparison, with about half of these being identical in all four sequences. Clusters of identical residues can be seen in three regions containing the conserved motifs L/VWV/AADLTYV, IHHT/SDRGSQY and C/SYDNA. The degree of conservation of these regions suggests that they are essential for the function of this protein.

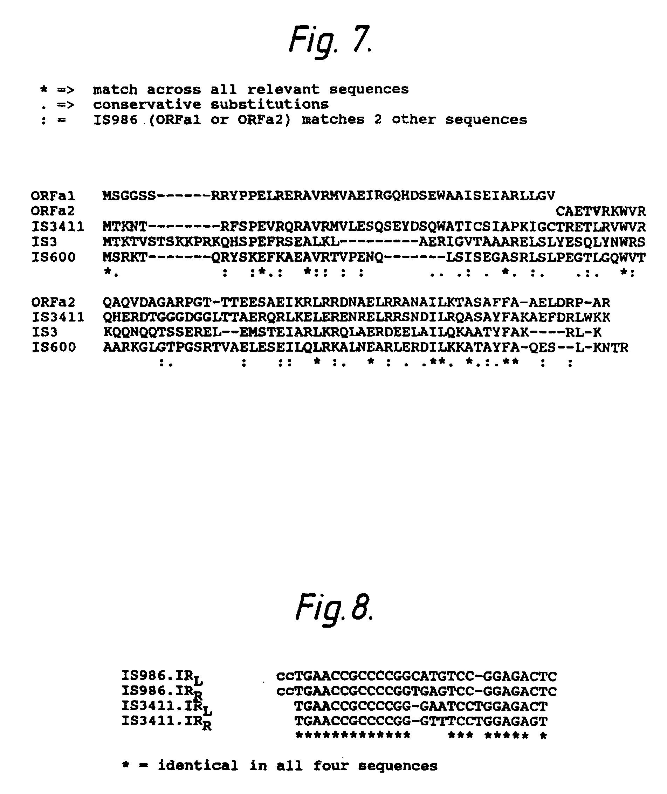

The sequence prior to the potential start codon in ORFb (GUG₄₇₈) bears only a weak resemblance to a consensus Shine-Dalgarno sequence, which is probably not significant. Therefore the nature of the potential translation start of ORFb was investigated by examination of the upstream region. The three-frame comparison of the translation products of IS3, IS3411 and IS986 indicated further similarities in this region. In both IS3 and IS3411, the putative transposase gene (ORFb) is preceded by an open reading frame of about 300 base pairs, with good translational start signals (Ishiguro, N. & Sato, G. (1988) Journal of Bacteriology 170, 1902-1906; and Matsutani, S., Ohtsubo, H., Maeda, Y. & Ohtsubo, E. (1987) Journal of Molecular Biology 196, 445-455). The hypothetical products of the relevant regions of these ORFs align well with those of ORFa1 and ORFa (Fig. 7) (the translated products of ORFa1 and ORFa2, up to and starting from the position of the suggested frameshift, were aligned with the products of the corresponding reading frame of the other elements. All sequences shown, except ORFa2, started from the presumed AUG initiation codon) indicating a possible frameshift in the IS986 sequence. Alternatively, there is a potential start codon (GUG₂₀₀) five amino acids into the sequence shown in Fig. 7; so it is conceivable that ORFa2 is translated independently. Of the combined ORFa1 and ORFa2 products, 29% of residues are found in two of the other three sequences shown. Pairwise comparisons confirm the alignments; for example, 50% of the residues match with the IS3411 ORFa product. The alignment shown in Fig. 7 is in marked contrast to the finding of Schwartz et al (Schwartz, E., Kroger, M. & Rak, B. (1988) Nucleic Acids Research 13, 2127-2139) that the ORFa products of several elements of the IS3 family showed only marginal homology.

The IS986 ORFa1 has a potential initiation codon (AUG) at position 54, preceded by a purinerich region with several possible assignments of sequences showing five out of seven bases matching the consensus Shine-Dalgarno sequence. With several other members of the IS3 family, translation of the putative transposase (ORFb) is thought to occur by readthrough from ORFa. In both IS3411 and IS3, the translational stop signal ending ORFa overlaps the putative start codon for ORFb, with the sequence AUGA. A ribosome terminating at this point could therefore reinitiate at the overlapping AUG codon. However, in IS986, although ORFa2 overlaps ORFb, there is no potential start codon in the overlapping region of ORFb.

Ribosomal frameshifting, generating a fusion protein, has been shown to occur in IS1 (Sekine, Y. & Ohtsubo, E. (1989) Proceedings of the National Academy of Sciences USA 86, 4609-4613) in a region where two ORFs overlap, probably at the sequence UUUAAAAAC. IS3411, IS3 and IS600 all contain runs of 5-7 A residues in the overlap region between the two ORFs. The overlap region between ORFa2 and ORFb in IS986 does not contain such a long run of adenines, but the sequence UUUUAAAG (324-331) may be a candidate for such a frameshifting event. Translational frameshifting in the -1 direction also occurs in other prokaryotic genes which do not appear to possess a common sequence (Atkins, J.F. Gesteland, R.F., Reid, B.R. & Anderson, C.W. (1979) Cell 18, 1119-1131).

The significance of ORFc, on the reverse strand, is unclear. The first potential start codon (AUG₁₀₀₂) is not preceded by anything resembling a Shine-Dalgarno sequence. Although analysis of ORFc is consistent with it being a translated sequence, it is in register with ORFb on the other strand, and the analytical procedures on the two strands are not independent. Schwartz et al (Schwartz, E., Kroger, M. & Rak, B. (1988) Nucleic Acids Research 14, 6789-6802) have identified a similar ORF in the E.coli element IS150, which appears to have a coding function. The presence of ORFs on the reverse strand is a common feature of other IS elements, and is considered to be involved in the regulation of transposition possibly by the requirement for both proteins ensuring that the IS element cannot be gratuitously activated by external transcription (Galas, D.J. and Chandler, M. (1989) in Mobile DNA (Berg, D.E. and Howe, M.M., Eds.), pp. 109-162. American Society for Microbiology. Washington). Further work is required to define the actual nature of the translational (and transcriptional) control signals operating in M.tuberculosis.

The base composition of IS986 is typical of M.tuberculosis, at 64% G+C. It is therefore not surprising that the homology with the other members of the IS3 family, which is so pronounced at the protein level, is much less striking at the DNA level (data not shown). There is however a marked degree of similarity of the inverted repeat ends with the other members of the IS3 family, especially IS3411 (Fig. 8) where the IR ends are 78% identical to those of IS986.

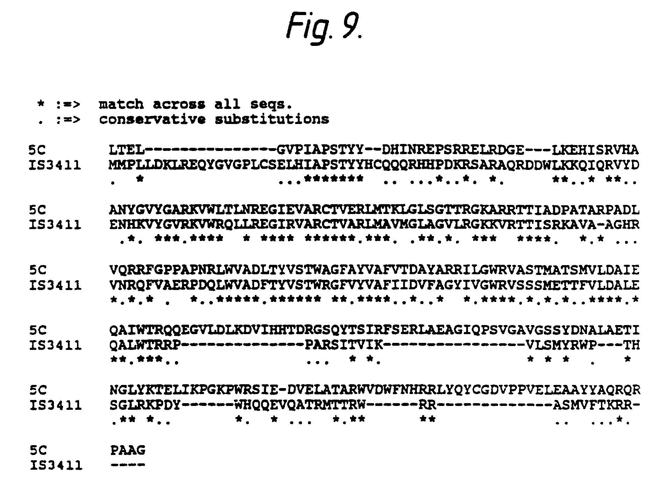

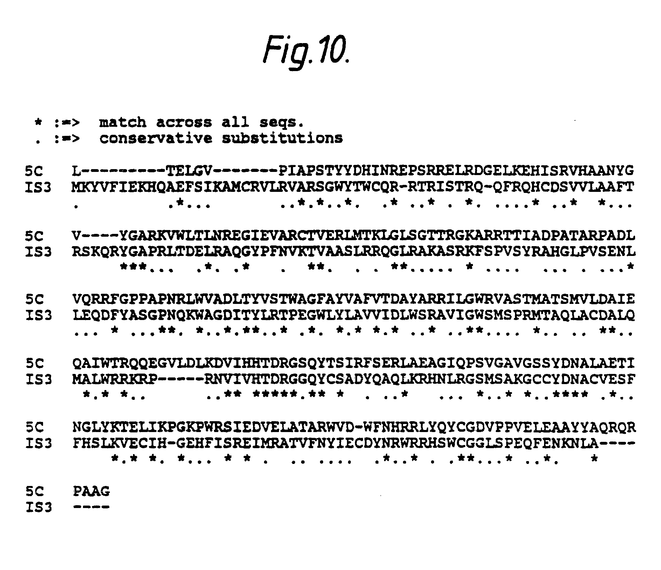

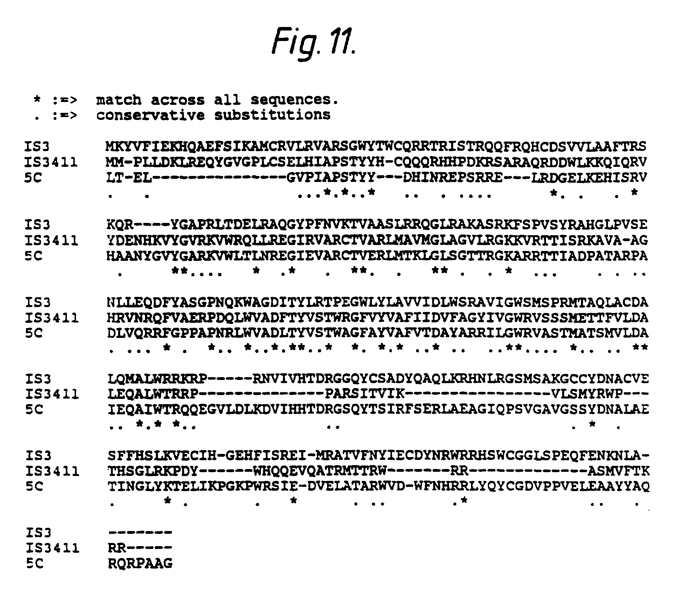

Fig. 9 shows that the translation of the large open reading frame from 5C is strongly homologous to the large open reading frame of insertion element IS3411 from E.coli. It is also homologous to IS3 from E.coli (Fig. 10). The alignment of all three sequences is shown in Fig. 11.

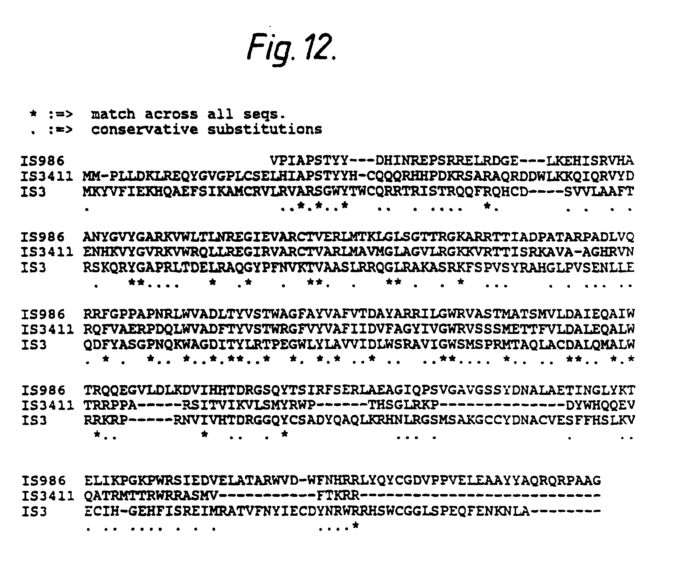

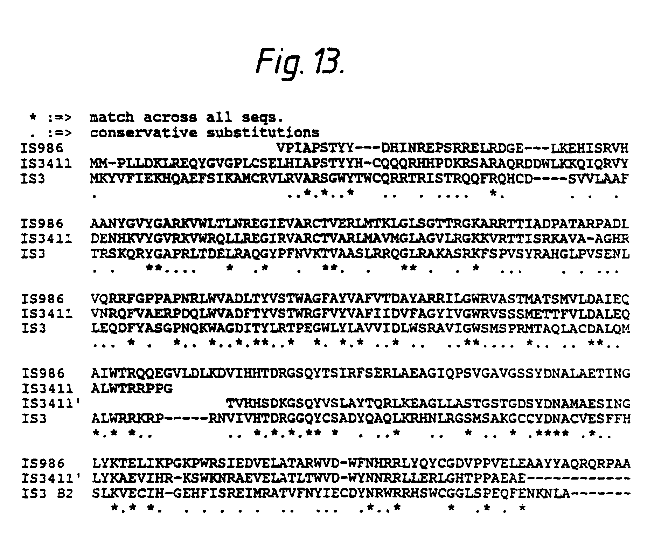

The alignment of the potential translated products of the large open reading frames of the insertion sequence from the 5kb DNA fragment of M.tuberculosis (IS986) with those of IS3411 and IS3 is shown in Fig. 12. In Fig. 13 a similar comparison is made, but here the C-terminal region of the IS3411 sequence (IS3411') is read from the - 1 frame with respect to the rest of the IS3411 sequence.

Probe 5 was tested by hybridisation experiments substantially as described for probe 9 with 22 isolates of M.tuberculosis as well as M.bovis and BCG. The conditions were the same as described above for probe 9, except that autoradiography was for 6.5 hours at room temperature.

Each M.tuberculosis strain showed between five and fifteen strongly hybridizing fragments, as well as a number of weaker bands. The number of bands and the strength of the signal, as well as the variation between strains, indicated the presence of a randomly inserted repetitive DNA element in the chromosome of these strains.

M.bovis and BCG showed a simpler pattern of two and three major bands respectively. These organisms could therefore be easily distinguished from M.tuberculosis and from each other.

The following species of mycobacteria (one strain each except where indicated) did not hybridize with probe 5: M.paratuberculosis, M.intracellulare, M.scrofulaceum, M.phlei, M.fortuitum (three strains) M.kansasii, M.avium, M.malnioense, M.flavescens, M.gordonae and M.chelonei (two strains).

Probe 5 was therefore, specific for the M.tuberculosis complex and was in addition able to distinguish between M.tuberculosis, M.bovis and BCG, and to distinguish between strains of M.tuberculosis

Fragment 5A on Southern blot, hybridises strongly and specifically with DNA from M.tuberculosis H₃₇Rv and H₃₇Ra and M.bovis BCG giving identical bands in each, of size 2.1 and 0.65 kbp, although it does not necessarily give these sized bands with any strain of M.tuberculosis.

### INDUSTRIAL APPLICABILITY

Part or all of the sequences identified and which comprise part or all of probe 5 can be used as gene probes. In particular, part or all of the sequences identified in 5C and 5B, as constituting the insertion element can be used as gene probes. When such probes are used in hybridisation studies on cleaved genomic DNA from bacterial specimens of the M.tuberculosis complex, characteristic banding patterns are produced and therefore such probes are useful as diagnostic and epidemiological tools. Not only different species, but different strains within a species produce characteristic banding patterns. This is particularly useful for distinguishing M.bovis and M.bovis BCG from other species, and indeed M.bovis from M.bovis BCG. Probe 5A could be used as a generic probe, for detecting all members of the M.tuberculosis complex.

The usefulness of probe 5 or a fragment thereof as a diagnostic tool is largely due to the following features.
a) The insertion element has only been found in members of the M.tuberculosis complex (M.tuberculosis, M.bovis, M.africanum and M.microti) and not in non-pathogenic environmental Mycobacteria nor M.leprae.
b) Using Southern blot analysis with probe 5 (or a part of the insertion element in 5) as a probe, a different pattern of bands is seen with each M.tuberculosis isolate tested (22 to date). This would be a powerful tool in epidemiological studies to examine tuberculosis transmission.
c) It is one of the first probes to show differences between M.tuberculosis and M.bovis and perhaps more importantly between M.bovis and M.bovis BCG.
d) The use of the insertion element as a probe to distinguish M.bovis BCG from M.bovis and M.tuberculosis is useful in patients with disseminated BCG infection following vaccination or immunosuppression.
e) Insertion elements (flanked by two insertion sequences) are useful genetic tools in characterising unknown genes.

Thus, we provide herein a number of ways of distinguishing and characterising bacterial members of the M.tuberculosis complex, both from each other and from other bacteria not of the complex.

For example, DNA from a sample of bacteria can be digested with a particular restriction enzyme and a hybridisation analysis carried out (in accordance with standard techniques) using as a probe a fragment of the DNA disclosed herein, which fragment does not contain the restriction site used to cleave the sample DNA. For example, a BamHI to Xho I fragment (or a part thereof) of probe 5/5C (see Fig. 1 and bases 420 to 810 of Fig. 2) which is located within the insertion element and which does not contain any PvuII sites, was used to probe a PvuII digest of M.bovis BCG DNA. When this was done, strong hybridisation to a single band was observed, indicating that in the M.bovis BCG strain tested, the insertion element is present in a single copy.

Employing a probe which contains the restriction site used to cleave the sample DNA, will give rise to multiple band hybridisation, as will also occur if the sample DNA contains multiple copies of e.g. the insertion element; as appears to be the case with most members of the M.tuberculosis complex. Nevertheless, the banding hybridisation patterns can be used to distinguish between different strains of the same species, and between different species of the M.tuberculosis complex. A generic probe for detecting all members of the M.tuberculosis complex need not include DNA from the insertion sequence, but could be exclusively from the flanking DNA, such as PvuII-EcoRI fragment 5A, as discussed above.

The existence in M.tuberculosis of an insertion sequence so closely related to characterised IS elements from the Enterobacteriaceae is of considerable significance from several points of view. The multiple restriction fragment length polymorphisms detected (Zainuddin, Z.F. & Dale, J.W. (1989) Journal of General Microbiology 135, 2347-2355) indicate that a number of copies of IS986 are inserted at different sites in different isolates of M.tuberculosis. In this respect it differs from other recently described repetitive elements from mycobacteria (Clark-Curtiss, J.E. & Walsh, G.P. (1989) Journal of Bacteriology 171, 4844-4851; Clark-Curtiss, J.E. & Docherty, M.A. (1989) Journal of Infectious Diseases 159, 7-15; and Green, E.P., Tizard, M.L. V., Moss, M.T., Thompson, J., Winterbourne, D.J., McFadden, J.J. & Hermon-Taylor, J. (1989) Nucleic Acids Research 17, 9063-9072) which give identical Southern blot patterns with different isolates. This suggests that IS986 may be a functional transposable element in mycobacteria, which would be of considerable value for transposon mutagenesis of mycobacterial species. The transposability of IS986 may be regulated by ribosomal frameshifting in the overlap between ORFa and ORFb, as has been established for IS1 (Sekine, Y. & Ohtsubo, E. (1989) Proceedings of the National Academy of Sciences USA 86, 4609-4613).

The presence of IS986 in clinically isolated strains of M.tuberculosis from a wide variety of sources (Zainuddin, Z.F. & Dale, J.W. (1989) Journal of General Microbiology 135, 2347-2355) and the relationship with the IS elements from E.coli and Sh.sonnei, suggest the possibility of transfer of genetic material amongst M.tuberculosis strains as well as acquisition from Gram negative bacteria. It has been suggested (Zainuddin, Z.F. & Dale, J.W. (1990) Tubercle 71, in press) that at least some clinical strains of M.tuberculosis carry plasmids, and these may play a role in the dissemination of such elements; the ability of some E.coli plasmids to replicate in Mycobacteria (Zainuddin, Z., Kunze, Z. & Dale, J.W. (1989) Molecular Microbiology, 29-34) may have enabled insertion sequences to spread from E.coli to M.tuberculosis. However, conjugation has never been conclusively demonstrated in M.tuberculosis, and the organism normally has a solitary existence, apart from incidental encounters with other organisms, e.g., in the gut. Therefore, transmission of plasmids carrying insertion sequences would probably be a rare event. The high G+C composition of the IS element indicates that its acquisition by M.tuberculosis is not a recent event. These questions may be resolved by a study of the behaviour of this insertion sequence in laboratory strains and clinical isolates.

IS986 is found in all species of the M.tuberculosis complex, although the copy number varies, and is not found in other mycobacterial species (Zainuddin, Z.F. & Dale, J.W. (1989) Journal of General Microbiology 135, 2347-2355). Therefore, probes based on IS986 will be highly specific for pathogenic mycobacteria. Coupled with the use of the Polymerase Chain Reaction (PCR), this will provide an exceptionally sensitive system for the detection and speciation of M.tuberculosis in clinical specimens. The extensive polymorphism of M.tuberculosis isolates tested with this probe (Zainuddin, Z.F. & Dale, J.W. (1989) Journal of General Microbiology 135, 2347-2355) enables extremely precise epidemiological investigations to be carried out, by fingerprinting clinical isolates. With this system all but the most closely related isolates will yield different patterns of hybridising restriction fragments, and it will thus be possible to track the spread of different strains of M.tuberculosis through a community.

### Probe 12

"Probe 12" is an Eco RI fragment of around 25.2 Kb from M.tuberculosis NCTC 7416 H₃₇Rv, obtained by screening a library of EcoRI - digested H₃₇Rv under stringent conditions, with H₃₇Rv DNA and isolating a strongly hybridising clone.

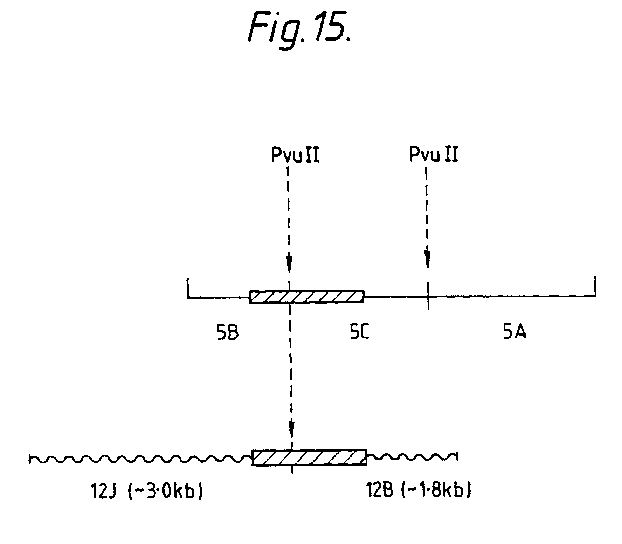

The 25.2 kb EcoRI fragment is digested by Pvull into fragments of approximate size 8.9 kb, 3.8 kb, 3.5 kb, 3.0 kb (fragment 12J), 1.8 kb (fragment 12B), 1.6 kb, 1.4 kb, and 1.2 kb (fragment 12A). The 1.2kb 12A fragment is M.tuberculosis complex specific and not related to probes 5 or 9. Figure 15 shows the arrangement of the 12J and 12B fragments with respect to probe 5. The DNA flanking the insertion sequence is illustrated by a wavy line as it is not identical to the flanking DNA in probe 5, owing to the fact that the insertion element inserts at many places in the genome. The flanking DNA of probe 12J hybridises with many different species of Mycobacteria. Fragment 12J could have value as a diagnostic probe for detecting a wide range of Mycobacteria.

### Probe 8

This describes an Eco RV fragment of approximately 16.1kb isolated by hybridisation screening a Eco RV library of H₃₇Rv.

When used as a probe on Southern blot with DNA from M.tuberculosis it binds to many fragments. On PvuII digestion it yields fragments of approximate size 5.6 kb, 4.8 kb, 2.1 kb, 2.0 kb, 0.9 kb and 0.7 kb. It does not appear to be related to probes 5 and 12.

## Claims

1. A nucleotide probe for the diagnosis and/or epidemiological study of Mycobacterial infection, which hybridizes with Mycobacterium tuberculosis genomic DNA obtainable by screening a Mycobacterium tuberculosis genomic library with DNA of a plasmid pUS300 of Mycobacterium fortuitum which nucleotide probe in hybridization assay is capable of distinguishing and characterising bacterial members of the Mycobacterium complex either from each other, or from other bacteria not of the complex and wherein the probe is other than said plasmid.

2. A nucleotide probe for the diagnosis and/or epidemiological study of Mycobacterial infection, which hybridizes with genomic DNA of Mycobacterium tuberculosis and with a plasmid pUS300 of Mycobacterium fortuitum which nucleotide probe in hybridization assay is capable of distinguishing and characterising bacterial members of the Mycobacterium complex either from each other, or from other bacteria not of the complex and wherein the probe is other than said plasmid.

3. A nucleotide probe according to claim 1, wherein the probe comprises a nucleotide sequence which comprises part of said DNA obtainable by screening, and which hybridises with a repetitive insertion element in the chromosome of Mvcobacterium tuberculosis strains.

4. A nucleotide probe according to claim 1 wherein the genomic library is obtained from Mycobacterium tuberculosis strain 50410.

5. A nucleotide probe for the diagnosis and/or epidemiological study of Mycobacterial infection which comprises, or hybridizes with part or all of a flanking sequence of nucleotides which, in the genome of Mycobacterium tuberculosis strain 50410 occur adjacent to an insertion element nucleotide sequence which occurs upstream of the 5' end of base 896 in Fig. 2 of the drawings.

6. A nucleotide probe for the diagnosis and/or epidemiological study of Mycobacterial infection which comprises, or hybridizes with, part or all of the flanking sequence of nucleotides which occurs downstream of the 3' end of base 896 in Fig. 2 of the drawings.

7. A nucleotide probe for the diagnosis and/or epidemiological study of Mycobacterial infection, which comprises, or hybridizes, with, part or all of an approximately 1.9kb nucleotide sequence which, in the genome of Mycobacterium tuberculosis strain 50410, occurs immediately downstream of the 3' end of the nucleotide sequence shown in Fig. 2 of the drawings.

8. A nucleotide probe according to any one of claims 1 to 5 which can distinguish between Mycobacterium tuberculosis, Mycobacterium bovis and BCG.

9. A nucleotide probe according to any one of claims 1 to 5 which can distinguish between different strains or isolates of Mycobacterium tuberculosis.

10. A nucleotide probe according to any one of claims 1 to 5 which does not show significant hybridization to nucleic acids from Mycobacterium paratuberculosis, Mycobacterium intracellulare, Mycobacterium phlei, Mycobacterium fortuitum and Mycobacterium malmoense.

11. A kit which comprises a nucleotide probe according to any one of claims 1 to 10.

12. A method for detecting, distinguishing and/or characterising Mycobacteria in clinical samples for the purposes of epidemiological study which comprises using a nucleotide probe according to any one of claims 1 to 5.

13. A method for distinguishing and characterising bacterial members of the Mycobacterium tuberculosis complex, either from each other, or from other bacteria not of the complex which comprises:
digesting DNA from a sample of bacteria with a particular restriction enzyme; and
carrying out hybridization analysis using a nucleotide probe according to any one of claims 1 to 5.

## Patentansprüche

1. Nukleotidsonde zur Diagnose und/oder epidemiologischen Untersuchung mycobakterieller Infektion, die mit genomischer Mycobacterium-tuberculosis-DNA hybridisiert, die durch das Screenen einer genomischen Mycobacterium-tuberculosis-Bibliothek mit DNA eines Plasmids pUS300 von Mycobacterium fortuitum erhältlich ist, wobei die Nukleotidsonde beim Hybridisierungstest fähig ist, bakterielle Bestandteile des Mycobacterium-Komplexes entweder voneinander oder von anderen Bakterien, die nicht zum Komplex gehören, zu unterscheiden und zu charakterisieren, und worin die Sonde eine andere als das Plasmid ist.

2. Nukleotidsonde zur Diagnose und/oder epidemiologischen Untersuchung mycobakterieller Infektion, die mit genomischer DNA von Mycobacterium tuberculosis und mit einem Plasmid pUS300 von Mycobacterium fortuitum hybridisiert, wobei die Nukleotidsonde beim Hybridisierungstest fähig ist, bakterielle Bestandteile des Mycobacterium-Komplexes entweder voneinander oder von anderen Bakterien, die nicht zum Komplex gehören, zu unterscheiden und zu charakterisieren, und worin die Sonde eine andere als das Plasmid ist.

3. Nukleotidsonde nach Anspruch 1, worin die Sonde eine Nukleotidsequenz umfasst, die einen Teil der durch Screenen erhältlichen DNA umfasst, und die mit einem repetitiven tnsertionsetement im Chromosom von Mycobacterium-tuberculosis-Stämmen hybridisiert.

4. Nukleotidsonde nach Anspruch 1, worin die genomische Bibliothek von Mycobacterium-tuberculosis-Stamm 50410 erhalten wird.

5. Nukleotidsonde zur Diagnose und/oder epidemiologischen Untersuchung von mycobakterieller Infektion, die einen Teil der oder die gesamte flankierende Sequenz von Nukleotiden umfasst oder damit hybridisiert, die im Genom von Mycobacteriumtuberculosis-Stamm 50410 angrenzend an eine Insertionselementnukleotidsequenz auftreten, die stromauf vom 5'-Ende der Base 896 in Fig. 2 der Zeichnungen auftritt.

6. Nukleotidsonde zur Diagnose und/oder epidemiologischen Untersuchung von mycobakterieller Infektion, die einen Teil oder die gesamte flankierende Sequenz von Nukleotiden umfasst oder damit hybridisiert, die stromab vom 3'-Ende der Base 896 in Fig. 2 der Zeichnungen auftritt.

7. Nukleotidsonde zur Diagnose und/oder epidemiologischen Untersuchung von mycobakterieller Infektion, welche einen Teil der oder die Gesamtheit einer Nukleotidsequenz mit in etwa 1,9 kb umfasst oder damit hybridisiert, die im Genom von Mycobacterium-tuberculosis-Stamm 50410 unmittelbar stromab vom 3'-Ende der in Fig. 2 der Zeichnungen gezeigten Nukleotidsequenz auftritt.

8. Nukleotidsequenz nach einem der Ansprüche 1 bis 5, die zwischen Mycobacterium tuberculosis, Mycobacterium bovis und BCG unterscheiden kann.

9. Nukleotidsequenz nach einem der Ansprüche 1 bis 5, die zwischen verschiedenen Stämmen oder Isolaten von Mycobacterium tuberculosis unterscheiden kann.

10. Nukleotidsonde nach einem der Ansprüche 1 bis 5, die keine wesentliche Hybridisierung an Nukleinsäuren von Mycobakterium paratuberculosis, Mycobakterium intracellulare, Mycobacterium phlei, Mycobacterium fortuitum und Mycobacterium malmoense zeigt.

11. Set, das eine Nukleotidsonde nach einem der Ansprüche 1 bis 10 umfasst.

12. Verfahren zum Nachweisen, Unterscheiden und/oder Charakterisieren von Mycobacteria in klinischen Proben zum Zweck epidemiologischer Untersuchung, welches die Verwendung einer Nukleotidsonde nach einem der Ansprüche 1 bis 5 umfasst.

13. Verfahren, um bakterielle Bestandteile des Mycobacterium-tuberculosis-Komplexes entweder voneinander oder von anderen Bakterien, die nicht zum Komplex gehören, zu unterscheiden und zu charakterisieren, umfassend:
Verdau von DNA von einer Bakterienprobe mit einem bestimmten Restriktionsenzym; und
die Durchführung von Hybridisierungsanalyse unter Verwendung einer Nukleotidsonde nach einem der Ansprüche 1 bis 5.

## Revendications

1. Sonde nucléotide pour le diagnostic et/ou l'étude épidémiologique d'infection myobactérienne, qui hybride avec l'ADN génomique Mycobacterium tuberculosis que l'on peut obtenir par tri d'une bibliothèque génomique de Mycobacterium tuberculosis avec un ADN d'un plasmide pUS300 de Mycobacterium fortuitum, laquelle sonde nucléotide dans un test d'hybridation est capable de distinguer et de caractériser les membres bactériens du complexe Mycobacterium, soit l'un de l'autre, soit d'autres bactéries qui ne sont pas du complexe et dans laquelle la sonde est différente dudit plasmide.

2. Sonde nucléotide pour le diagnostic et/ou l'étude épidémiologique d'infection mycobactérienne, qui hybride avec l'ADN génomique de Mycobacterium tuberculosis et a vc un plasmide pUS3àà de Mycobacterium fortuitum laquelle sonde nucléotide dans un test d'hybridation est capable de distinguer et de caractériser des membres bactériens du complexe Mycobacterium soit l'un de l'autre, soit d'autres bactéries qui ne sont pas du complexe et dans laquelle la sonde est différente dudit plasmide.

3. Sonde nucléotide selon la revendication 1, où la sonde comprend une séquence nucléotide qui comprend une partie dudit ADN que l'on peut obtenir par tri et qui hybride avec un élément respectif d'insertion dans le chromosome de souches de Mycobacterium tuberculosis.

4. Sonde nucléotide selon la revendication 1, dans laquelle la bibliothèque génomique est obtenue à partir d'une souche de Mycobacterium tuberculosis 50410.

5. Sonde nucléotide pour le diagnostic et/ou l'étude épidémiologique d'injection Mycobactérienne qui comprend, ou hybride avec une partie ou toute une séquence de flanc de nucléotides qui, dans le génome d'une souche de Mycobacterium tuberculosis 50410 se produit adjacente à une séquence nucléotide d'un élément d'insertion qui se produit en amont de l'extrémité 5' de base 896 sur la figure 2 des dessins.

6. Sonde nucléotide pour le diagnostic et/ou l'étude épidémiologique d'injection Mycobactérienne qui comprend, ou hybride avec une partie ou toute la séquence de flanc de nucléotides qui apparaît en aval de l'extrémité 3' de base 896 sur la figure 2 des dessins.

7. Sonde nucléotide pour le diagnostic et/ou l'étude épidémiologique d'injection Mycobactérienne qui comprend, ou hybride avec, une partie ou toute une séquence nucléotide d'approximativement 1,9 kb, qui dans le génome de la souche 50410 de Mycobacterium tuberculosis, apparaît immédiatement en aval de l'extrémité 3' de la séquence nucléotide montrée en figure 2 des dessins.

8. Sonde nucléotide selon l'une quelconque des revendications 1 à 5 qui peut distinguer entre du Mycobacterium tuberculosis, du Mycobacterium bovis et du BCG.

9. Sonde nucléotide selon l'une quelconque des revendications 1 à 5 qui peut distinguer entre différentes souches ou isole du Mycobacterium tuberculosis.

10. Sonde nucléotide selon l'une des revendications 1 à 8, qui ne montre pas d'hybridation significative aux acides nucléituqes provenant de Mycobacterium paratuberculosis, mycobacterium intracellulareMycobacterium phlei Mycobacterium fortuitium et Mycobacterium malnioense

11. Kit qui comprend une sonde nucléotide selon l'une quelconque des revendications 1 à 10.

12. Procédé pour détecter, distinguer et/ou caractériser des Mycobactéries dans des échantillons cliniques dans des buts d'étude épidémiologique qui comprend l'utilisation d'une sonde nucléotide selon l'une quelconque des revendications 1 à 5.

13. Procédé pour distinguer et caractériser des membres bactériens de complexe Mycobacterium tuberculosis, soit l'un de l'autre, soit d'autres bactéries qui ne sont pas du complexe qui comprend:
la digestion d'ADN à partir d'un échantillon de bactéries avec un enzyme de restriction particulier; et
la mise en oeuvre d'une analyse d'hybridation utilisant une sonde nucléotide selon l'une quelconque des revendications 1 à 5.
